# EUROPEAN PATENT SPECIFICATION

(11) **EP 3 429 983 B1**
(45) Date of publication and mention of the grant of the patent: **12.01.2022**
(21) Application number: 16893810.8
(22) Date of filing: 16.03.2016
(51) Int. Cl.: C07C 29/76, C07C 31/20, C09K 3/18, C07C 29/80

(54) **METHOD AND SYSTEM FOR RECYCLING SPENT ETHYLENE GLYCOL FROM RECOVERED AIRCRAFT DE-ICING SOLUTIONS**
VERFAHREN UND SYSTEM ZUM RECYCLING VON VERBRAUCHTEM ETHYLENGLYKOL AUS WIEDERGEWONNENEN ENTEISUNGSMITTELLÖSUNGEN EINES FLUGZEUGS
PROCÉDÉ ET SYSTÈME DE RECYCLAGE DE L'ÉTHYLÈNEGLYCOL USÉ RÉCUPÉRÉ DANS DES SOLUTIONS DE DÉGIVRAGE D'AÉRONEFS

(43) Date of publication of application: 23.01.2019
(73) Proprietor: Aéro Mag 2000 RRR Inc., St-Laurent, Québec H4S 2A5 (CA)
(72) Inventor: LÉPINE, Mario, Dorval, Québec H9S 2H4 (CA); BERGERON, Ghislain, Saint-Eustache, Québec J7P 0A3 (CA); GUY, Michel, Pierrefonds, Québec H9J 3R4 (CA)
(74) Representative: BCF Global
(86) International application number: PCT/CA2016/000074
(87) International publication number: WO 2017/156615

(56) References cited:
- WO-A2-2010/072311
- CA-A1- 2 116 827
- CA-A1- 2 915 300
- US-A- 5 904 321
- US-A1- 2011 303 870
- STEVE SMITH: 'A Tour of the Munich International Airport's Deicing Recycling Plant' AVIATIONPROS, [Online] 28 February 2012, XP055425872 Retrieved from the Internet: <URL:http://www.aviationpros.com/article/10 616425/a-tour-of-the- munich -international-airports-deici ng-recycling-plant> [retrieved on 2016-10-11]
- 'Montreal-Trudeau Airport, Aero Mag Inaugurates Deicing Fluid Recovery And Reuse Facility' AVIATIONPROS, [Online] 26 November 2014, XP055425902 Retrieved from the Internet: <URL:http://www.aviationpros.com/press _ release/12022687/montreal-trudeau-airport-a ero-mag-inaug urates-deicing-fluid-recoverv-and-reuse-fac ility> [retrieved on 2016-10-11]
- 'Cost Effective Airport Aircraft Deicing Fluid Management' THERMOENERGY CORPORATION, [Online] 05 November 2012, XP055425880 Retrieved from the Internet: <URL:http://content.stockpr.com/tmen/media/ 2bb16a93fc2d4657592d6b3f0497751e.pdf> [retrieved on 2016-10-11]

## Description

### TECHNICAL FIELD

The present invention relates to a method and a system for recycling spent ethylene or propylene glycol recovered from aircraft deicing solutions to produce virgin glycol having a concentration of at least 99.5% and up to 99.9%.

### BACKGROUND ART

It is a requisite to de-ice the accumulation of frost, ice and snow or combinations thereof on the wings of aircrafts to restore the natural shape of the wings by removing any frost, ice or snow build-up that distorts its shape and to permit proper functioning of the ailerons. Ice, frost or snow also adds to the weight of the aircraft, thereby impeding flight. The removal of frost, ice or snow is effected by spraying a de-icing glycol solution to cause the frost, ice or snow to melt away and protects the aircraft for sufficient time after de-icing against further precipitation. The concentration of glycol in the solution varies according to climatic conditions. Such solutions typically comprise glycol, water, and minor amounts of additives such as surfactants, and a corrosion inhibitor.

After spraying the de-icing solution on the aircraft, the solution becomes diluted with water from melted frost, ice and snow and becomes contaminated with pollutants present on the tarmac area where aircraft propagate to be de-iced. These areas are usually concrete areas polluted with sand, abraded rubber from aircraft tires, oil, combustion residue, fumes from the aircraft, deicing salts, grit, traces of fuel, solid debris such as grass and leaves, and chemicals found in concrete. In the past, such waste glycol solutions where harnessed in reservoirs or vacuum trucks for disposal and treatment at remote sites for disposal. Such a practice proved to be a burden on the environment and resulted in the loss of glycol which is an expensive product. Typically, the airport assumes the cost of this disposal and by recovering the spent glycol substantive savings is passed on to the airport facility.

Over the last decades, efforts have been made to recycle glycol residue from aircraft de-icing solutions and various recovery systems have been placed in operation using various methods such as aerobic digesters, cyclone separators, chemical cleaning techniques using absorption and ion exchangers, percolation towers, distillation and stripping columns or towers, etc. However, the operation of some of these have proven problematic and very costly and some have not achieved the desired sought after result of recovering glycol of sufficient purity comparable to purchased virgin glycol, for recycling for use in an aircraft deicing glycol solution. Some of the known techniques have claimed to recycle such glycol solutions to a purity of 99.5% but many have failed to produce this purity. Examples of known techniques can be found, for example, in the patent literature with reference to US Patents 5,904,321; 5,411,668; 7,713,319; 8,252, 149; US Patent Application Publications 2011/0263909 and 2013/0190539, as well as Canadian Patents 2, 116,827 and 2,223,922. Another publication on the topic of recycling glycol from airport tarmacs can be found in an article entitled "A Tour Of The Munich International Airport's Deicing Recycling Plant", such article being accessible on the web at http;//www.aviationpros.com/article/10616425. US2011/303870A1 discloses a process for workup of glycol-containing aircraft deicers. CA2915300A1 discloses a method for reprocessing aircraft deicing agents comprising glycol.

### SUMMARY OF THE INVENTION

There is a need to provide an improved method and a system, which is substantially automatic, for recycling spent ethylene or propylene glycol recovered from aircraft deicing solutions to produce virgin glycol having a concentration of at least 99.5% and typically in the order of from between 99.6% to 99.9%.

The invention is the method and system as defined in the claims.

Provided herein is a method of recycling spent ethylene or propylene glycol recovered from aircraft de-icing solutions containing glycol, water and other substances to produce substantially virgin glycol. The method comprises, in combination, the steps of recovering spent glycol from the deicing facilities of airports and storing the spent glycol in one or more storage tanks. Thereafter, the spent glycol having a predetermined low % concentration is removed to produce a working spent glycol and storing same in a storage means. The working spent glycol is filtered through at least two filtering stages to substantially remove all solids and other substances and stored in a holding tank. An evaporation step then removes water from a batch of filtered spent glycol by heating the batch to a temperature to evaporate water to bring the working spent glycol to a glycol concentration of about 50%. The glycol concentration solution is then stored in a buffer tank. The pH of the solution of glycol concentration of 50% is then adjusted to a desired value and then it is carbon filtered and fed to a holding tank which feeds a predetermined batch to an evaporator section of a distillation tower operating under vacuum where it is then heated to predetermined temperatures to evaporate the liquids in the glycol concentration of 50% in a stream of vapors. The temperature of the stream of vapor in a packing section of the distillation section of the tower is sensed to monitor the actual temperature of the stream of vapor and temperature signals representative thereof are sent to a computer controller. The stream of vapor at the top of the distillation tower is cooled as it exits from the packing section to condense into a liquid phase. The glycol concentration of the condensed liquid is monitored on a continuous basis to detect the glycol concentration of the condensed liquid and glycol concentration signals are sent to the computer controller which correlates these with the temperature signals to determine the appropriate time to operate valves to recover from the condensed liquid, in associated ones of reservoirs, water, water mixed with glycol below a concentration of 99.5%, and glycol solution of at least 99.5%having a concentration of at least 99.5%. The water, water mixed with glycol below a concentration of 99.5%, and the glycol solution of at least 99.5% is automatically directed to associated reservoirs. The glycol of at least 99.5% is tested and blended and directed to certified holding tanks for use by aircraft de-icing vehicles.

Moreover there is provided herein a system for recycling spent ethylene or propylene glycol recovered from aircraft deicing solutions containing glycol, water and other substances to produce substantially virgin glycol. The system comprises, in combination, collection means for recovering spent glycol from the tarmac of an aircraft de-icing area. Storage means is provided for storing the recovered spent glycol. Means is further provided to remove spent glycol having a predetermined low % glycol concentration from the storage means. A working tank is also provided for maintaining a predetermined volume of the spent glycol having a glycol concentration above the predetermined low % glycol concentration. At least two filter stages is also provided to filter the spent glycol from the working tank to remove substantially all solid particles from the other substances and to feed it to a holding tank to feed an evaporator stage having one or more evaporators to evaporate water from the batch of spent glycol solution by boiling the batch at a temperature sufficient to evaporate water to produce a spent glycol having a glycol concentration of about 50% which is then transferred to a buffer tank. Means is provided to adjust the pH of the batch of glycol concentration from the buffer tank to a desired pH value and it is then carbon filtered and fed to a distillation tower holding tank to accumulate a predetermined volume of the spent glycol concentration above about 50%. A distillation tower is provided and operates under vacuum and has a lower evaporator section and an upper chimney section provided with steel packings to retain heat from a stream of vapors released from the lower evaporator section. Temperature sensors monitor the temperature of the chimney section along the packings to provide actual temperature signals to the computer controller representative of the actual temperature to the stream of vapors drawn through the packings and provides glycol concentration value signals to the computer controller. The lower evaporator section has a temperature controlled heater to evaporate the spent glycol in sequence to create the stream of vapors whose vapors are drawn through the chimney section and into a condensing coil downstream of the chimney section to produce condensed liquid which is fed through a cooling device. Measuring means is further provided to measure, on a continuous basis, the glycol concentration in the condensed liquid, and provide glycol concentration values to the computer controller to correlate with the temperature signals to determine the appropriate time to operate valves to separate, in associated reservoirs, water, water mixed with glycol having a glycol concentration of less than 99.5% purity, and substantially virgin glycol having a glycol concentration of at least 99.5% up to 99.9%. Means is then provided to certify and store the substantially virgin glycol in certified holding tanks ready for blending for use by aircraft de-icing vehicles.

### BRIEF DESCRIPTION OF THE DRAWINGS

A preferred embodiment of the present invention will now be described with reference to the accompanying drawings in which;

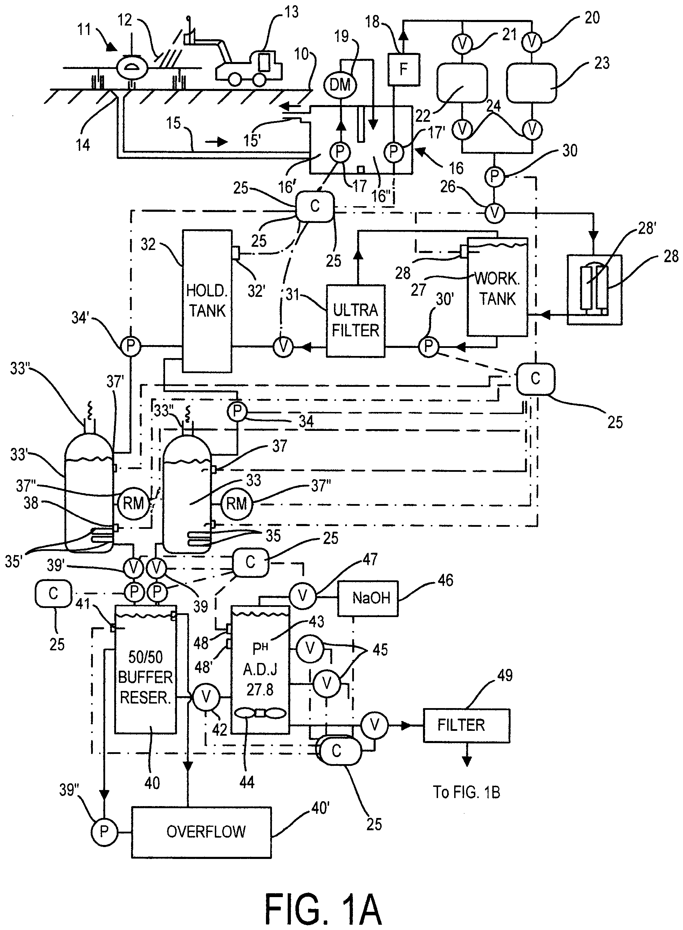

Figures 1A to 1C are schematic and partly block diagrams, illustrating the improved batch processing method and system of the present invention.

### DESCRIPTION OF THE PREFERRED EMBODIMENT

Referring to the drawings there will now be described a preferred embodiment of method and system of the present invention for recycling spent ethylene or propylene glycol recovered from aircraft deicing solutions. As shown in Fig. 1A, the spent ethylene glycol herein of Type I or type IV is recovered from the tarmac 10 of an airport where aircrafts, such as the one schematically illustrated at 11, are sprayed with deicing solution 12 from deicing vehicles 13. The deicing solution is usually heated to a temperature of 60 to 80 degrees C (centigrade). To recover spent glycol, the tarmac area is modified to be fitted with one or more storm drains 14 connected to underground conduits 15 to channel the spent glycol to a storage chamber 16 which is segmented. The spent glycol enters a first compartment 16' which also communicates with a larger chamber 16". The configuration of these chambers allows some sediments to precipitate at the bottom of chamber 16". A sample of spent glycol is continuously flowing through a densimeter 19 driven by pump 17 located in chamber 16' in order to assess the spent glycol concentration. If the concentration of the spent glycol is below 5%, the spent glycol is not suitable for recycling. Therefore, the computer controller 25 allows the level of spent glycol to gradually rise into chambers 16 until it reaches an overflow 15' to city sewage.

The computer controller 25 also operates a further pump 17' in the compartment 16" to pump solution of 5% or more concentration from the tank compartment 16" through a first filter 18 to remove sediments and feed the spent glycol of 5% or more concentration into holding tanks, herein only two tanks 22 and 23 being illustrated which are fitted with level detectors (not shown, but obvious to a person skilled in the art) to permit the computer controller to operate valves 21 and 20 to direct the spent glycol solution of 5% or more concentration to other holding tanks when a tank is full.

It is pointed out that the computer controller has various monitoring and control stations or different computers and such is identified throughout the drawings by the letter "C" inside a small square and reference numeral 25. It is also pointed out that throughout the drawings, the sizes of the squares, representing different units, tanks, filters, etc, are not representative of their size in proportion to one another.

From the storage tanks 22 and 23 the spent glycol solution of 5% or more concentration is treated substantially automatically by the computer controller 25, as follows by operating a valves 24 and a pump 30 spent glycol solution is subjected to a two stage filtering process. Firstly, the spent glycol is fed through a bag filter 28 and into a working tank 27 where a pump 30' circulates the filtered spent glycol concentration of 5% or more through an ultra fine filter 31, herein a ceramic filter, and back to the working tank 27 on a continuous basis. Ultra filtered spent glycol permeating through the ceramic filter by pressure is fed into holding tank 32 which has a level sensor 32' which when a predetermined volume of filtered spent glycol is reached , signals the computer controller to stop the pumps 30 and 30'. The bag filtering stage has tow bag filters 28 and 28' to provide for longer uninterrupted operation by switching over to the other bag when one of the bags becomes clog with sediments. This permits longer operational time of the system. The ultra fine ceramic filter 31 has a pore size of 0.5 microns to remove very fine solid particles leaving substantially only liquids, often referred to as permeated liquid.

The filtered solution from holding tank 32 is then pumped, by pumps 34 and pump 34' into two evaporators 33 and 33' of like constructions. Each evaporator 33, 33' is provided with two electric resistive heating elements 35 and 35', respectively, which are operated by the computer controller 25 to ensure continuous operation of the evaporators in the event of failure of one of the resistive heating elements. Although not shown, these known evaporators 33 and 33' are each provided with a compressor (not shown) to extract heat from the vapors released in their flue 33" and re-use the heat from the vapors as a principal source to heat the water in the reservoirs. The resistive heating elements 35 and 35' are used to pre-heat the water at start-up, permitting the evaporation of water at lower temperature under vacuum conditions.

Level sensors 37 transmit level indicating signals to the computer controller 25 to operate the pumps 34 and 34' to provide sufficient spent solution in the evaporator whereby to switch one of the heating element on, the other being on stand-by and placed in service by a switch not shown, but obvious, in the event of failure of the other resistive heating element.. Temperature sensing probes 38 provide actual temperature values to the computer controller of the batch solution in the evaporators 33 and 33'. The heating elements 35 and 35' are operated to control the temperature of the water to a boiling temperature of 100 degrees C sufficient to evaporate water, but too low for ethylene or propylene glycol to evaporate. Refractometers 37" detect the density of the spent solution at an appropriate level and feeds representative signals to the computer controller 25 to operate valves 39 and 39' once the concentration of glycol attains 50% concentration. Valves 39 and 39' are operated to transfer batches of the spent glycol of 50% concentration to a buffer tank 40 on a continuous basis. The evaporators 33 and 33' operate on a continuous basis and as the volume of spent glycol solution in holding tank 32 drops to a predetermined level the pumps 30 and 30' are again actuated to filter more spent solution to feed ultra filtered solution to the holding tank 32. Any overflow from the buffer tank 40 is collected in reservoirs 40' for future use. In this embodiment there are several reservoirs 40' capable of holding 3 million liters of the solution of 50% glycol concentration. A pump 39" supplies the solution to the buffer tank 40 when needed to maintain the process continuously operational. Also, the reservoirs 40 and 40' are maintained inside a building to manage heat loss from the spent glycol of 50% concentration to save energy costs. In the preferred embodiment herein described, the buffer tank holds 40,000 liters of the solution. An overflow valve, not shown, recovers any solution which exceeds the capacity of the tank and feeds.

After a desired volume, sensed by the signals from the level sensor 41, is present in the buffer tank 40, the computer controller 25 operates a transfer valve 42' and a pump, not shown but obvious, to transfer progressively some of the spent glycol solution over to a pH adjustment tank 43 provided with an agitator 44 for analysis as the volume of spent glycol solution in tank 43 is known as detected by level sensing probe 48 having informed the computer controller. The computer controller 25 also operates a transfer valve 47 and a pump, not shown but obvious, when the pH sensor 48', located in the tank 43, measures any low pH by amending the spent glycol solution with sodium hydroxide (NaOH) solution from reservoir 46. The pH is adjusted to a desired value of about 7.8 on the pH scale. The adjusted pH spent glycol solution of 50% concentration is then fed through a carbon filter 49 to remove color and odors from the spent glycol solution before being stored into a distillation tower feed tank 50 where a predetermined quantity of the pre-treated spent glycol is maintained and controlled by level sensors.

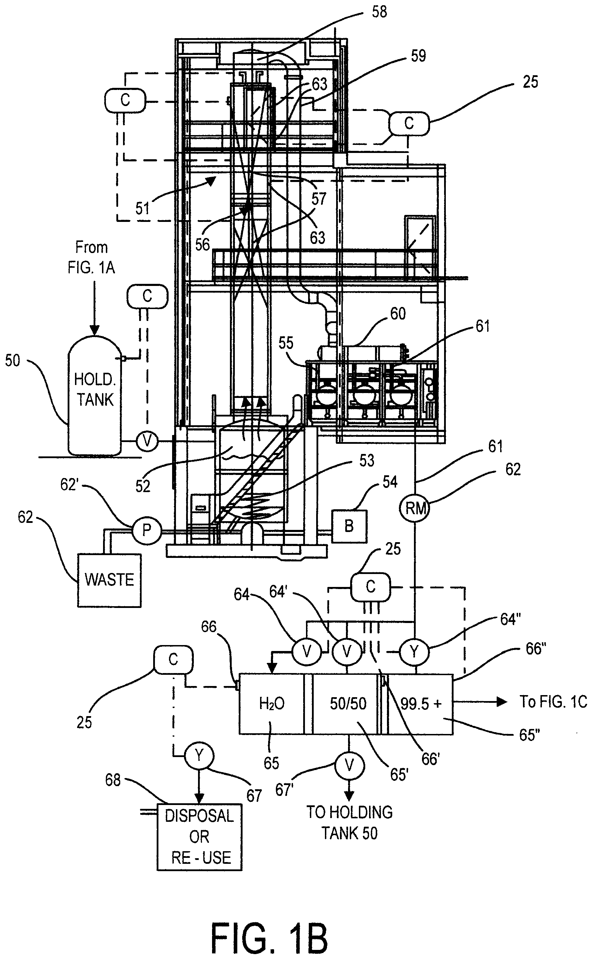

As shown in Fig. 1B, the process includes a distillation tower 51 which is essentially comprised of a bottom evaporator section 52 which includes a heating coil 53 in which is circulated, in a controlled manner, hot steam from a boiler 54 whereby to boil a predetermined batch of the spent glycol solution of 50% glycol concentration to evaporate substantially all of the liquid solution in the batch. Because the boiling temperature of water is far inferior to the boiling temperature of glycol, water will start evaporating as soon as the batch temperature reaches the boiling point of water. Accordingly, only water will evaporate at first, followed by water mixed with glycol, and lastly high concentration glycol. By controlling the flow rate of steam in the coil we can increase the temperature of the spent liquid in the evaporators section to evaporate the liquids in stages. Once all the liquids have evaporated, the sensed temperature in the packings will drop providing an indication that the entire batch is evaporated.

A vacuum pump 55 connects to the distillation tower 51 at an appropriate downstream location outside the tower to place the tower under vacuum. The distillation tower has a chimney section 56 above the evaporator section 52 and in which there is provided metal packings 57, herein stainless steel corrugated perforated sheets which accumulate heat from the hot stream of vapors released from the boiling liquid in the evaporator section. The temperature of the vapor passing through the packings is monitored by temperature sensors 63 feeding temperature signals 63' to the computer controller 25 to provide an indication of the temperature of the stream of steam giving an indication of its content in relation to its temperature. These temperature signals also indicate the start and the end of the evaporation cycle. The temperature sensors may be in the form of thermistors retained biased and spaced apart vertically against the wall of the chimney section. The distillation tower is also thermally insulated by a shroud spaced about the inner casing with an appropriate insulation material disposed there between, much like the insulated tank of a water heater. The boiler is heated with natural gas and circulates sufficiently high temperature steam through the heating coil 53 whereby the boiling water temperature rises substantially quickly to the boiling point of glycol which is about 197 degrees C which is far greater than the boiling point of water. The density of glycol is about 1.1132 g/cubic centimeter. It is pointed out that the vacuum conditions in the distillation tower decreases slightly the boiling points of each liquid to be evaporated, namely water and glycol, and this also provides an energy cost saving.

At the top of the chimney section 56 there is mounted a condenser 58 which condenses the stream of vapor as it exits the distillation tower 51 to turn the vapor into its liquid phase. The condensation is outside the chimney whereby the condensate does not fall back into the chimney section. As mentioned, because the water evaporates earlier than the glycol, at the beginning of the evaporation process, mostly water is condensed and the condensate with some vapor is channeled by a long conduit 59 where further condensation takes place and into a further cooler 60 where any residual vapor is condensed. Nothing is released into the atmosphere to prevent the loss of glycol. Secured to an outlet pipe 61 of the cooler 60 is a refractometer 62 which measure the glycol concentration of glycol which may be contained in the condensed liquid and feeds its readings to the computer controller 25, which in correlation with temperature signals 63 received from the chimney section, determines the proper time to operate valves 64 associated with a respective one of three reservoirs or tanks 65, 65' and 65".

When only water is detected, valve 64 is open and the other two valves 64' and 64" are closed thereby to channel the water into reservoir 65 for disposal or re-use. When computer controller 25 determines that the condensate liquid contains glycol mixed with water, the computer controller 25 closes valves 64 and 64" and opens valve 64' to channel the mixture into reservoir 65' until the computer controller determines from its correlated signals that the glycol concentration about 99.5%. The computer controller then closes the valves 64 and 64' and opens valve 64" to channel the remaining glycol which is classified as virgin glycol into reservoir 65". Preferably, the condensed liquid is switched over to reservoir 65" when the computer controller 25 receives condensate signals indicating 99.6% to 99.9% glycol concentration in the solution from refractometer 63 which is classified as virgin glycol. Level sensors 66, 66' and 66" provide signals to the computer controller indicating the volume of liquid in its associated reservoirs whereby to evacuate its contents to appropriate locations. Valve 67 dispenses the recovered water for appropriate storage in tank 68 for disposal or re-use. Valve 67' permits its content to be transferred back into the holding tank 50 to be re-introduced into the distillation tower. Finally, residual material consisting of high boiling point compounds collect in the sump lower section of the evaporator section and is pumped into a waste tank 62 at the end of the evaporation cycle and another batch is introduced for evaporation and separation.

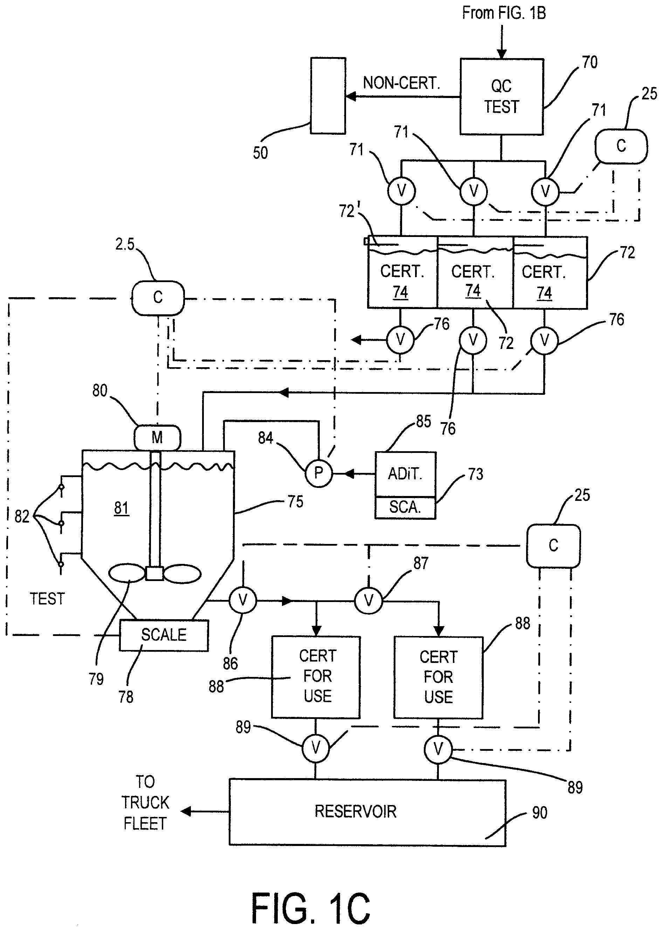

Referring now to Fig. 1C, it can be seen that the glycol solution batch from reservoir 65" is now subjected to a further quality control testing in a further reservoir 70 to certify its glycol concentration by measuring the Fisher methodology, the Brix and testing of the pH. Sampling valves (not shown) are provided at various levels of the reservoir 70 for the extraction of samples for lab analysis. After certification, the computer controller 25 operates valves 71 to transfer the certified glycol into certified reservoirs 72. The contents of the reservoir 72 is monitored by level sensors 72' feeding information signals to the computer controller 25 whereby the content can be safely transferred and to indicate that the valves 71 need to be operated to switch the transfer of solutions to other reservoirs 72, 72' and 72". With the batch process of the present invention the concentration of the recycled glycol in the solution can be brought up to 99.9% whereby it achieves a very high level of purity which is very important to provide a final solution of near perfect concentration, the requisite for virgin glycol of the highest purity to assure for a perfect blended deicing solution for re-use.

At this point in the process, the certified glycol solution 74 in one or several reservoirs 72 need to receive further final additives and this is effected in a blending tank 75. The solution 74 is transferred into the blending tank by the computer controller 25 operating selected ones of transfer valves 76 until the blending tank is filled with a predetermined weight as sensed by a scale 78 mounted under the blending tank 75. Once the scale 78 indicates that the blending tank has received its volume of glycol solution 74, the computer controller 25 shuts off the valve(s) 76. At this point ADF blending is done by the addition of additives 85, also sensed by a scale 73, which are introduced into the solution content 81 by operating pump 84 to bring the fluid solution to a desired concentration, for example 88%. An agitator 79 is operated by switching on its motor 80 to mix the glycol solution content 81. Sampling valves 82 permit sampling the content 81 after the agitating cycle to effect tests, testing the pH, and the refracting index to assure that the content 81 can be certified as a type I de-icing glycol. Valves 86 and 87, controlled by the computer controller 25 direct the final adjusted certified solution to additional reservoirs 88 equipped with valves 89 for dispensation to farm tank 90 at a location accessible to aircraft deicing vehicles. These vehicles effect online blending of the deicing glycol solution by adding water which may have been recovered in the process, depending on the outside temperature, to adjust the glycol concentration.

The present invention is intended to cover any obvious modifications of the preferred embodiment described, provided such modifications fall within the scope of the appended claims.

## Claims

1. A method of recycling spent ethylene or propylene glycol recovered from aircraft de-icing solutions (12) containing glycol, water and other substances to produce substantially virgin glycol, said method comprising, in combination, the steps of:
i) recovering spent glycol (12) from the de-icing facilities of airports and storing same in storage means,
ii) removing (15') spent glycol having a predetermined low % purity concentration from spent glycol stored in said storage means to produce a working spent glycol and storing said working spent glycol in one or more storage tanks (22,23),
iii) filtering said working spent glycol from said one or more storage tanks (22,23) through at least two filtering stages (28,31) to substantially remove all solids in said other substances followed by an evaporation step (33,33') wherein said working spent glycol is heated to a temperature sufficient to evaporate only water to bring said working spent glycol to a glycol concentration of about 50% purity for storage in a buffer tank (40),
iv) adjusting the pH of said glycol concentration of about 50% in an agitator tank to adjust the pH to a desired value and then carbon filtering said glycol concentration of about 5%,
v) feeding a predetermined volume of said carbon filtered glycol concentration of about 50% in an evaporator section of a distillation tower operating under vacuum,
vi) heating said batch at a predetermined temperature to evaporate said glycol concentration of about 50% in a continuous stream of vapors,
vii) sensing the temperature of said stream of vapors in a hot packing section of a chimney section spaced above said distillation section to monitor the actual temperature of said stream of steam and feeding temperature signals representative thereof to a computer controller,
viii) cooling said stream of vapors after it exits said chimney section to condense said stream of vapors into a condensed liquid,
ix) monitoring on a continuous basis (a) said temperature signals to correlate same with the evaporation temperature of water and glycol, and (b) glycol concentration signals form a refractometer located in a conduit through which flows said condensed liquid, to recover from said condensed liquid, water, water mixed with glycol below a concentration of 99.5%, and substantially virgin glycol having a concentration of at least 99.5%, and
x) directing said substantially virgin glycol from its associated reservoir for further testing and certification of the purity thereof and storing same in certified holding tanks for use by aircraft de-icing vehicles.

2. The method of claim 1 wherein after step (x) of certification of the purity thereof and storing same in certified holding tanks in step (x), there is provided the further step of blending said certified glycol solution of at least 99.5% into a blending tank where additives are blended with said certified substantially virgin glycol.

3. The method of claim1 wherein said substantially virgin glycol of step (ix) has a concentration in the range of at least 99.5% up to 99.9%.

4. The method of claim 1 wherein in step (ix), said separated water mixed with glycol below a concentration of 99.5% and collected in associated reservoirs is fed back to a holding tank of said distillation tower to be re-introduced into said distillation tower for further evaporation, said step (v) further comprising feeding said predetermined volume of carbon filtered glycol concentration in said holding tank of said distillation tower.

5. The method of claim 1 wherein in said step (ii) said spent glycol having a predetermined low concentration of below 5%, as determined by a densimeter which feeds glycol concentration signals to a computer controller, is caused to rise in said storage means to an overflow conduit for disposal to sewage.

6. The method of claim 1 wherein in said filtering step (iii) one of said at least two filtering stages comprises a 5 micron bag filter which feeds filtered glycol solution to a working tank and wherein said filtered glycol solution from said working tank is pumped in a continuous loop and through a 0.5 micron ceramic filter, said working spent glycol being filtered in said continuous loop to achieve a desired filtered spent glycol free of substances of particle size above 0.5 microns, and feeding said ultra-filtered spent glycol to a further holding tank which feeds said filtered spent glycol to one or more evaporators.

7. The method of claim 6 wherein said further holding tank is provided with level sensing means to provide level signals to a computer controller which controls the operation of said continuous filtering loop, said evaporators being operated on a continuous basis and having level sensing means to permit said computer controller to dispense filtered spent glycol solution thereto to maintain a predetermined volume of said filtered spent glycol solution in said one or more evaporators ..

8. The method of claim 7 wherein said evaporator is provided with two electric resistive heating elements connected for independent operation through switch means operated by said computer controller to ensure continuous operation of said evaporator in the event of failure of one of said resistive heating elements, a densimeter associated with each said one or more evaporators to detect the glycol concentration in a predetermined area of said evaporators to feed glycol concentration signal values to said computer controller to operate valves to discharge spent glycol solution having a concentration of 50% from a bottom region of said one or more evaporators into said buffer tank and to shut off said valve when said concentration signal values fall below 50% glycol concentration.

9. The method of claim 1 wherein said glycol concentration of about 50% stored in said buffer tank is subjected to a pre-treatment as defined by step (iv) by the addition of NaOH (sodium hydroxide) in said agitator tank as desired to adjust the pH to said desired value, said desired value being about 7.8 on the pH scale, and further wherein said carbon filtering of step (v) is to remove color and odors from said glycol concentration before feeding same to a holding tank of said distillation tower where a control level of said pre-treated glycol concentration of 50% is maintained for batch feeding an evaporator section of said distillation tower.

10. The method of claim 1 wherein said evaporator section of said distillation tower has a heating coil in which is circulated hot steam from a boiler to evaporate in said stream of vapors said predetermined batch of glycol concentration of 50%, a vacuum pump connected to said distillation tower downstream of said evaporator section, said hot packing section having a plurality of stainless steel packings which are heated along said chimney section by said stream of vapors, and wherein after said step (viii) and before said step (ix) there is provided the further step of feeding said condensed liquid into a cooler device to achieve complete condensation.

11. The method of claim 10 wherein said step (ix) of monitoring, is effected by a refractometer sending signals to said computer controller which operates valves to direct said condensed liquids to said associated ones of said reservoirs.

12. The method of claim 2 wherein said blending tank is provided with level tap valves to permit testing of the pH, the clarity, the Brix and refracting index of said substantially virgin de-icing glycol at different levels of said blending tank, the volume of said substantially virgin glycol in said blending tank being determined by a scale disposed under said blending tank, and pumping means to pump substantially virgin glycol from said blending tank to said certified holding tanks.

13. A system for recycling spent ethylene or propylene glycol recovered from aircraft deicing solutions containing glycol, water and other substances to produce substantially virgin glycol, said system comprises, in combination, a collection means for recovering spent glycol from the tarmac of an aircraft de-icing area, means is further provided to remove spent glycol having a predetermined low % concentration from said collection means, one or more working tanks are also provided for storing spent glycol having a glycol concentration above said predetermined low% concentration, filter means is also provided to filter the spent glycol from the working tank to remove substantially all solids from said spent glycol, said filter means having at least two filtering stages one of which is a loop ultra-filtering means, one or more evaporators to evaporate water from said spent glycol to produce a spent glycol having a glycol concentration of about 50% which is then transferred in batches to a buffer tank, means is provided to adjust the pH of the batch of glycol concentration of about 50% to a desired pH value which is then carbon filtered and fed to a distillation tower holding tank to accumulate a predetermined volume of the spent glycol concentration above about 50% concentration to feed a batch of spent glycol to an evaporator section of a distillation tower which operates under vacuum, an upper chimney section is provided with stainless steel packings to retain heat above condensation temperatures of the evaporated liquid from said lower evaporator section, temperature sensors monitoring the temperature of said chimney section provided with said packings to feed detected temperature signals representative of the temperature of a stream of vapors drawn through said packings and feeding said temperature signals to a computer controller, said lower evaporator section evaporates the spent glycol to create said stream of vapors whose vapors are drawn through the chimney section and into a condensing coil downstream of said chimney section to produce condensed liquid from said stream of vapors which is fed through a cooling device; measuring means is further provided to measure on a continuous basis the glycol concentration in the condensed liquid and feed glycol concentration signals to said computer controller to correlate with said temperature signals to determine the appropriate time to operate valves whereby to separate, in associated reservoirs, water, water mixed with glycol having a glycol, and substantially virgin glycol having a glycol concentration of at least 99.5%, and quality testing means to certify and store said substantially virgin glycol in certified holding tanks for use by aircraft deicing vehicles.

14. The system of claim 13 wherein said means to remove spent glycol having a predetermined low % concentration is constituted by an overflow conduit in an upper portion of said collection means, and a pump adapted to pump spent glycol from a predetermined region of said collection means to a densimeter to feed spent glycol concentration signals to a computer controller which determines if spent glycol requires to be discharged into said overflow conduit or if spent glycol having a concentration of at least 5% should be transferred to said one or more working tanks.

15. The system of claim 13 wherein said filter means includes a first filter stage to produce a first stage filtered batch of spent glycol for collection in a working tank, a second ultra filtering stage secured in a loop through which filtered spent glycol from said working tank is continuously circulated to extract fine filtered spent glycol to feed an evaporator holding tank.

16. The system of claim 15 wherein said second ultra-filtering stage includes an ultra-fine ceramic filter through which said fist stage filtered batch of spent glycol from said holding tank is fed to remove further solids from said other substances above 0.5 microns.

17. The system of claim 15 wherein there is further provided blending means to incorporate additives in said substantially virgin glycol to produce type I deicing glycol of the highest quality and above at least 99.5% concentration.

18. The system of claim 17 wherein there is further provided a pH adjusting mixing tank having sampling means to test the pH of said spent glycol of about 50% glycol concentration and means to introduce additives to said mixing tank to produce a desired pH value for said spent glycol of about 50% glycol concentration prior to transferring said spent glycol of about 50% glycol concentration to a further holding tank to feed batches of said 50% spent glycol concentration into said lower evaporator section of said distillation tower.

19. The system of claim 13 wherein said separated substantially virgin glycol concentration of at least 99.5% is preferably in the range of from about 996% to 99.9% concentration.

20. The system of claim 13 wherein said measuring means to monitor the percentage concentration of glycol is effected by a refractometer to measure the refractive indexof said glycol in said condensate liquid, said refractometer providing reflective index value signals to a computer controller.

21. The system of claim 20 wherein said separation means are valves which are automatically operated by said computer controller based on received index value signals from said refractometer whereby to operate said valves to direct said condensate liquid to said associated ones of storage reservoirs.

22. The system of claim 21 wherein said storage reservoirs are provided with conduit means to direct said collected condensate liquid through a valve to a desired location with said water in one of said storage reservoir being directed for disposal or re-use, said water mixed with glycol below a concentration of 99.5% being directed back to said further holding tank feeding said evaporator section of said distillation tower, and said substantially virgin glycol being directed to certified glycol storage tanks.

23. The system of claim 22 wherein there is further provided a quality control testing holding tank to provide for the quality control testing for certification of said substantially virgin glycol, and a mixing tank for adding further additives to said certified substantially virgin glycol for storage in tanks ready for use by vehicles adapted for blending a final aircraft de-icing glycol solution depending on climatic factors.

## Patentansprüche

1. Verfahren zum Recycling von verbrauchtem Ethylen- oder Propylenglykol, das aus Enteisungsmittellösungen (12) eines Flugzeugs wiedergewonnen wird, die Glykol, Wasser und andere Substanzen enthalten, um im Wesentlichen natives Glykol herzustellen, wobei das Verfahren in Kombination die folgenden Schritte umfasst:
i) Wiedergewinnen von verbrauchtem Glykol (12) aus den Enteisungsanlagen von Flughäfen und Lagern desselben in Lagereinrichtungen,
ii) Entfernen (15') von verbrauchtem Glykol mit einer vorbestimmten niedrigen prozentualen Reinheitskonzentration aus dem in der Lagereinrichtung gelagerten verbrauchten Glykol, um ein verbrauchtes Arbeitsglykol zu erzeugen, und Lagern des verbrauchten Arbeitsglykols in einem oder mehreren Lagertanks (22, 23),
iii) Filtern des verbrauchten Arbeitsglykols aus dem einen oder den mehreren Lagertanks (22, 23) durch mindestens zwei Filterstufen (28, 31), um im Wesentlichen alle Feststoffe aus den anderen Substanzen zu entfernen, gefolgt von einem Verdampfungsschritt (33, 33'), wobei das verbrauchte Arbeitsglykol auf eine Temperatur erhitzt wird, die ausreicht, um nur Wasser zu verdampfen, um das verbrauchte Arbeitsglykol auf eine Glykolkonzentration von etwa 50 % Reinheit für die Lagerung in einem Puffertank (40) zu bringen,
iv) Einstellen des pH-Wertes der Glykolkonzentration von etwa 50 % in einem Rührwerkstank, um den pH-Wert auf einen gewünschten Wert einzustellen, und anschließendes Aktivkohlefiltern der Glykolkonzentration von etwa 5 %,
v) Zuführen eines vorbestimmten Volumens der aktivkohlefiltrierten Glykolkonzentration von etwa 50 % in einen Verdampferabschnitt eines unter Vakuum betriebenen Destillationsturms,
vi) Erhitzen der Charge auf eine vorbestimmte Temperatur, um die Glykolkonzentration von etwa 50 % in einem kontinuierlichen Dampfstrom zu verdampfen,
vii) Erfassen der Temperatur des Dampfstroms in einem Heißpackungsabschnitt eines Kaminabschnitts, der über dem Destillationsabschnitt angeordnet ist, um die tatsächliche Temperatur des Wasserdampfstroms zu überwachen, und Zuführen von Temperatursignalen, die dafür repräsentativ sind, zu einer Computersteuerung,
viii) Abkühlen des Dampfstroms, nachdem er den Kaminabschnitt verlassen hat, um den Dampfstrom zu einer kondensierten Flüssigkeit zu kondensieren,
ix) kontinuierliches Überwachen (a) der Temperatursignale, um sie mit der Verdampfungstemperatur von Wasser und Glykol zu korrelieren, und (b) der Glykolkonzentrationssignale aus einem Refraktometer, das in einer Leitung angeordnet ist, durch die die kondensierte Flüssigkeit fließt, um aus der kondensierten Flüssigkeit Wasser, Wasser gemischt mit Glykol unter einer Konzentration von 99,5 % und im Wesentlichen natives Glykol mit einer Konzentration von mindestens 99,5 % wiederzugewinnen, und
x) Leiten des im Wesentlichen nativen Glykols aus seinem zugehörigen Behälter zur weiteren Prüfung und Zertifizierung seiner Reinheit und Lagern desselben in zertifizierten Vorratstanks zur Verwendung durch Flugzeugenteisungsfahrzeuge.

2. Verfahren nach Anspruch 1, wobei nach dem Schritt (x) der Zertifizierung seiner Reinheit und des Lagerns derselben in zertifizierten Vorratstanks in Schritt (x) der weitere Schritt des Mischens der zertifizierten Glykollösung von mindestens 99,5 % in einem Mischtank vorgesehen ist, in dem Zusatzstoffe mit dem zertifizierten im Wesentlichen nativen Glykol gemischt werden.

3. Verfahren nach Anspruch 1, wobei das im Wesentlichen native Glykol aus Schritt (ix) eine Konzentration im Bereich von mindestens 99,5 % bis 99,9 % aufweist.

4. Verfahren nach Anspruch 1, wobei in Schritt (ix) das separierte Wasser, das mit Glykol unter einer Konzentration von 99,5 % gemischt und in zugehörigen Behältern aufgefangen wurde, zu einem Vorratstank des Destillationsturms zurückgeführt wird, um zur weiteren Verdampfung wieder in den Destillationsturm eingeführt zu werden, wobei der Schritt (v) ferner das Zuführen des vorbestimmten Volumens der aktivkohlefiltrierten Glykolkonzentration in den Vorratstank des Destillationsturms umfasst.

5. Verfahren nach Anspruch 1, wobei in Schritt (ii) das verbrauchte Glykol, das eine vorbestimmte niedrige Konzentration von unter 5 % aufweist, wie durch einen Dichtemesser bestimmt, der Glykolkonzentrationssignale einer Computersteuerung zuführt, veranlasst wird, in der Lagereinrichtung zu einer Überlaufleitung zur Entsorgung in das Abwasser aufzusteigen.

6. Verfahren nach Anspruch 1, wobei in dem Filterschritt (iii) eine der mindestens zwei Filterstufen einen 5-Mikrometer-Beutelfilter umfasst, der filtrierte Glykollösung einem Arbeitstank zuführt, und wobei die filtrierte Glykollösung aus dem Arbeitstank in einer kontinuierlichen Schleife und durch einen 0,5-Mikrometer-Keramikfilter gepumpt wird, wobei das verbrauchte Arbeitsglykol in der kontinuierlichen Schleife filtriert wird, um ein gewünschtes filtriertes verbrauchtes Glykol frei von Substanzen mit einer Partikelgröße über 0,5 Mikrometer zu erhalten, und das ultrafiltrierte verbrauchte Glykol einem weiteren Vorratstank zugeführt wird, der das filtrierte verbrauchte Glykol einem oder mehreren Verdampfern zuführt.

7. Verfahren nach Anspruch 6, wobei der weitere Vorratstank mit Füllstandsmesseinrichtungen bereitgestellt ist, um einer Computersteuerung, die den Betrieb der kontinuierlichen Filterschleife steuert, Füllstandssignale bereitzustellen, wobei die Verdampfer kontinuierlich betrieben werden und Füllstandsmesseinrichtungen aufweisen, die es der Computersteuerung ermöglichen, filtrierte verbrauchte Glykollösung darauf abzugeben, um ein vorbestimmtes Volumen der filtrierten verbrauchten Glykollösung in dem einen oder den mehreren Verdampfern aufrechtzuerhalten.

8. Verfahren nach Anspruch 7, wobei der Verdampfer mit zwei elektrischen Widerstandsheizelementen, die für einen unabhängigen Betrieb durch Schaltereinrichtungen verbunden sind, die durch die Computersteuerung betrieben werden, um einen kontinuierlichen Betrieb des Verdampfers im Falle des Ausfalls eines der Widerstandsheizelemente sicherzustellen, einem Dichtemesser, der jedem der einen oder mehreren Verdampfern zugeordnet ist, um die Glykolkonzentration in einem vorbestimmten Bereich der Verdampfer zu erfassen, um Glykolkonzentrationssignalwerte der Computersteuerung zuzuführen, um Ventile zu betätigen, um verbrauchte Glykollösung mit einer Konzentration von 50 % aus einer Bodenregion des einen oder der mehreren Verdampfer in den Puffertank abzulassen und das Ventil zu schließen, wenn die Konzentrationssignalwerte unter 50 % Glykolkonzentration sinken, bereitgestellt ist.

9. Verfahren nach Anspruch 1, wobei die Glykolkonzentration von etwa 50 %, die in dem Puffertank gespeichert ist, einer Vorbehandlung gemäß Schritt (iv) unterzogen wird, indem NaOH (Natriumhydroxid) in dem Rührwerkstank nach Bedarf zugesetzt wird, um den pH-Wert auf den gewünschten Wert einzustellen, wobei der gewünschte Wert etwa 7,8 auf der pH-Skala beträgt, und ferner wobei das Aktivkohlefiltern aus Schritt (v) darin besteht, Farbe und Gerüche aus der Glykolkonzentration zu entfernen, bevor sie einem Vorratstank des Destillationsturms zugeführt wird, wo ein Steuerniveau der vorbehandelten Glykolkonzentration von 50 % für die Chargenzufuhr eines Verdampferabschnitts des Destillationsturms aufrechterhalten wird.

10. Verfahren nach Anspruch 1, wobei der Verdampferabschnitt des Destillationsturms eine Heizwendel aufweist, in der heißer Dampf aus einem Kessel zirkuliert wird, um in dem Dampfstrom die vorbestimmte Charge einer Glykolkonzentration von 50 % zu verdampfen, wobei eine Vakuumpumpe stromabwärts des Verdampferabschnitts mit dem Destillationsturm verbunden ist, wobei der Heißpackungsabschnitt eine Vielzahl von Edelstahlpackungen aufweist, die entlang des Kaminabschnitts durch den Dampfstrom erhitzt wird, und wobei nach dem Schritt (viii) und vor dem Schritt (ix) der weitere Schritt bereitgestellt ist, die kondensierte Flüssigkeit einer Kühlvorrichtung zuzuführen, um eine vollständige Kondensation zu erreichen.

11. Verfahren nach Anspruch 10, wobei der Schritt (ix) des Überwachens dadurch erfolgt, dass ein Refraktometer Signale an die Computersteuerung sendet, die Ventile betätigt, um die kondensierten Flüssigkeiten zu den zugehörigen einen der Behälter zu leiten.

12. Verfahren nach Anspruch 2, wobei der Mischtank mit Füllstandszapfventilen bereitgestellt ist, um eine Prüfung des pH-Werts, der Klarheit, des Brix-Werts und des Brechungsindex des im Wesentlichen nativen Enteisungsglykols bei verschiedenen Füllständen des Mischtanks zu ermöglichen, wobei das Volumen des im Wesentlichen nativen Glykols in dem Mischtank durch eine unter dem Mischtank angeordnete Waage und Pumpeinrichtungen zum Pumpen von im Wesentlichen nativen Glykols aus dem Mischtank in die zertifizierten Vorratstanks bestimmt wird.

13. System zum Recycling von verbrauchtem Ethylen- oder Propylenglykol, das aus Enteisungslösungen eines Flugzeugs wiedergewonnen wird, die Glykol, Wasser und andere Substanzen enthalten, um im Wesentlichen natives Glykol herzustellen, wobei das System in Kombination eine Auffangeinrichtung zur Rückgewinnung von verbrauchtem Glykol von dem Asphalt eines Flugzeugenteisungsbereichs umfasst, wobei ferner Einrichtungen bereitgestellt sind, um verbrauchtes Glykol mit einer vorbestimmten niedrigen prozentualen Konzentration aus diesen Auffangeinrichtungen zu entfernen, ein oder mehrere Arbeitstanks zur Lagerung von verbrauchtem Glykol mit einer Glykolkonzentration oberhalb der vorbestimmten niedrigen prozentualen Konzentration ebenfalls bereitgestellt sind, Filtereinrichtungen ebenfalls bereitgestellt sind, um das verbrauchte Glykol aus dem Arbeitstank zu filtrieren, um im Wesentlichen alle Feststoffe aus dem verbrauchten Glykol zu entfernen, wobei die Filtereinrichtung mindestens zwei Filterstufen, von denen eine eine Schleifen-Ultrafiltereinrichtung ist, einen oder mehrere Verdampfer, um Wasser aus dem verbrauchten Glykol zu verdampfen, um ein verbrauchtes Glykol mit einer Glykolkonzentration von etwa 50 % herzustellen, das dann in Chargen in einen Puffertank überführt wird, aufweist, wobei Einrichtungen bereitgestellt sind, um den pH-Wert der Charge mit einer Glykolkonzentration von etwa 50 % auf einen gewünschten pH-Wert einzustellen, die dann aktivkohlefiltriert und einem Vorratstank des Destillationsturms zugeführt wird, um ein vorbestimmtes Volumen der verbrauchten Glykolkonzentration über der Konzentration von etwa 50 % anzusammeln, um eine Charge von verbrauchtem Glykol einem Verdampferabschnitt eines Destillationsturms zuzuführen, der unter Vakuum betrieben wird, wobei ein oberer Kaminabschnitt mit Edelstahlpackungen bereitgestellt ist, um Wärme über Kondensationstemperaturen der verdampften Flüssigkeit aus dem unteren Verdampferabschnitt zu halten, wobei Temperatursensoren, die die Temperatur des mit den Packungen bereitgestellten Kaminabschnitts überwachen, um erfasste Temperatursignale zuzuführen, die für die Temperatur eines durch die Packungen gezogenen Dampfstroms repräsentativ sind, und Zuführen der Temperatursignale an eine Computersteuerung, wobei der untere Verdampferabschnitt das verbrauchte Glykol verdampft, um den Dampfstrom zu erzeugen, dessen Dämpfe durch den Kaminabschnitt und in eine Kondensationswendel stromabwärts des Kaminabschnitts gezogen werden, um kondensierte Flüssigkeit aus dem Dampfstrom herzustellen, die durch eine Kühlvorrichtung geleitet wird; ferner sind Messeinrichtungen bereitgestellt, um kontinuierlich die Glykolkonzentration in der kondensierten Flüssigkeit zu messen und Glykolkonzentrationssignale der Computersteuerung zuzuführen, um sie mit den Temperatursignalen zu korrelieren, um den geeigneten Zeitpunkt für das Betätigen von Ventilen zu bestimmen, wodurch in zugehörigen Behältern Wasser, mit Glykol vermischtes Wasser mit einem Glykol und im Wesentlichen natives Glykol mit einer Glykolkonzentration von mindestens 99,5 % separiert werden, und Qualitätsprüfeinrichtungen, um das im Wesentlichen native Glykol zu zertifizieren und in zertifizierten Vorratstanks zur Verwendung durch Flugzeugenteisungsfahrzeuge zu lagern.

14. System nach Anspruch 13, wobei die Einrichtung zum Entfernen von verbrauchtem Glykol mit einer vorbestimmten niedrigen prozentualen Konzentration durch eine Überlaufleitung in einem oberen Bereich der Auffangeinrichtung und eine Pumpe gebildet ist, die angepasst ist, um verbrauchtes Glykol aus einer vorbestimmten Region der Auffangeinrichtung zu einem Dichtemesser zu pumpen, um Konzentrationssignale des verbrauchten Glykols einer Computersteuerung zuzuführen, die bestimmt, ob verbrauchtes Glykol in die Überlaufleitung abgelassen werden muss oder ob verbrauchtes Glykol mit einer Konzentration von mindestens 5 % in den einen oder die mehreren Arbeitstanks überführt werden soll.

15. System nach Anspruch 13, wobei die Filtereinrichtung eine erste Filterstufe zur Erzeugung einer ersten filtrierten Charge von verbrauchtem Glykol zum Auffangen in einem Arbeitstank und eine zweite Ultrafilterstufe beinhaltet, die in einer Schleife befestigt ist, durch die filtriertes verbrauchtes Glykol aus dem Arbeitstank kontinuierlich zirkuliert wird, um feinfiltriertes verbrauchtes Glykol zum Zuführen an einen Verdampfervorratstank zu extrahieren.

16. System nach Anspruch 15, wobei die zweite Ultrafiltrierstufe einen ultrafeinen Keramikfilter beinhaltet, durch den durch die erste Stufe filtrierte Charge verbrauchten Glykols aus dem Vorratstank zugeführt wird, um weitere Feststoffe aus den anderen Substanzen über 0,5 Mikrometer zu entfernen.

17. System nach Anspruch 15, wobei ferner Mischeinrichtungen bereitgestellt sind, um Zusatzstoffe in das im Wesentlichen native Glykol zu integrieren, um Enteisungsglykol vom Typ I von höchster Qualität und über einer Konzentration von mindestens 99,5 % herzustellen.

18. System nach Anspruch 17, wobei ferner ein pH-einstellender Mischtank mit Probenahmeeinrichtungen zum Prüfen des pH-Werts des verbrauchten Glykols mit etwa 50 % Glykolkonzentration und Einrichtungen zum Einbringen von Zusatzstoffen in den Mischtank bereitgestellt sind, um einen gewünschten pH-Wert für das verbrauchte Glykol mit etwa 50 % Glykolkonzentration herzustellen, bevor das verbrauchte Glykol mit etwa 50 % Glykolkonzentration in einen weiteren Vorratstank überführt wird, um Chargen des verbrauchten Glykols mit einer Konzentration von 50 % dem unteren Verdampferabschnitt des Destillationsturms zuzuführen.

19. System nach Anspruch 13, wobei die separierte im Wesentlichen native Glykolkonzentration von mindestens 99,5 % vorzugsweise im Bereich von etwa 99,6 % bis 99,9 % Konzentration liegt.

20. System nach Anspruch 13, wobei die Messeinrichtung zum Überwachen der prozentualen Konzentration von Glykol durch ein Refraktometer zur Messung des Brechungsindex des Glykols in der Kondensatflüssigkeit gebildet wird, wobei das Refraktometer Brechungsindexwertsignale einer Computersteuerung bereitstellt.

21. System nach Anspruch 20, wobei die Separationseinrichtungen Ventile sind, die durch die Computersteuerung basierend auf den von dem Refraktometer empfangenen Indexwertsignalen automatisch betätigt werden, um dadurch Ventile zu betätigen, um die Kondensatflüssigkeit zu den zugehörigen Speicherbehältern zu leiten.

22. System nach Anspruch 21, wobei die Lagerbehälter mit Leiteinrichtungen bereitgestellt sind, um die aufgefangene Kondensatflüssigkeit durch ein Ventil an einen gewünschten Ort zu leiten, wobei das Wasser in einem der Lagerbehälter zur Entsorgung oder Wiederverwendung geleitet wird, wobei das mit Glykol unter einer Konzentration von 99,5 % vermischte Wasser zurück in den weiteren Vorratstank geleitet wird, der den Verdampferabschnitt des Destillationsturms speist, und das im Wesentlichen native Glykol in zertifizierte Glykollagertanks geleitet wird.

23. System nach Anspruch 22, wobei ferner ein Qualitätskontrollprüf-Vorratstank zum Bereitstellen der Qualitätskontrollprüfung für die Zertifizierung des im Wesentlichen nativen Glykols und ein Mischtank zum Zusetzen weiterer Zusatzstoffe zu dem zertifizierten im Wesentlichen nativen Glykol zur Lagerung in Tanks, die für die Verwendung durch Fahrzeuge geeignet sind, die zum Mischen einer endgültigen Flugzeugenteisungsglykollösung in Abhängigkeit von klimatischen Faktoren angepasst sind, bereitgestellt sind.

## Revendications

1. Procédé de recyclage d'éthylène glycol ou de propylène glycol usé récupéré à partir de solutions de dégivrage d'aéronef (12) contenant du glycol, de l'eau et d'autres substances pour produire du glycol sensiblement vierge, ledit procédé comprenant, en combinaison, les étapes de :
i) récupération du glycol usé (12) à partir des installations de dégivrage des aéroports et stockage de celui-ci dans un moyen de stockage,
ii) élimination (15') du glycol usé ayant une concentration en pourcentage de pureté faible prédéterminée du glycol usé stocké dans ledit moyen de stockage pour produire du glycol usé fonctionnel et stockage dudit glycol usé fonctionnel dans une ou plusieurs cuves de stockage (22, 23),
iii) filtration dudit glycol usé fonctionnel desdites une ou plusieurs cuves de stockage (22, 23) à travers au moins deux étages de filtration (28, 31) pour éliminer sensiblement tous les solides dans lesdites autres substances suivie d'une étape d'évaporation (33, 33') dans laquelle ledit glycol usé fonctionnel est chauffé à une température suffisante pour évaporer uniquement l'eau afin d'amener ledit glycol usé fonctionnel à un glycol de concentration d'environ 50 % de pureté pour le stockage dans une cuve tampon (40),
iv) ajustement du pH dudit glycol de concentration d'environ 50 % dans une cuve d'agitateur pour ajuster le pH à une valeur souhaitée et ensuite filtration sur charbon dudit glycol de concentration d'environ 5 %,
v) fourniture d'un volume prédéterminé dudit glycol filtré sur charbon de concentration d'environ 50 % à une section d'évaporateur d'une tour de distillation fonctionnant sous vide,
vi) chauffage dudit lot à une température prédéterminée pour évaporer ledit glycol de concentration d'environ 50 % dans un flux continu de vapeurs,
vii) détection de la température dudit flux de vapeurs dans une section de remplissage chaud d'une section de cheminée espacée au-dessus de ladite section de distillation pour surveiller la température réelle dudit flux de vapeurs et fourniture de signaux de température représentatifs de celle-ci à un dispositif de commande informatique,
viii) refroidissement dudit flux de vapeurs après sa sortie de ladite section de cheminée pour condenser ledit flux de vapeurs en un liquide condensé,
ix) surveillance en continu (a) desdits signaux de température pour les corréler avec la température d'évaporation de l'eau et du glycol, et (b) des signaux de concentration de glycol à partir d'un réfractomètre situé dans un conduit à travers lequel s'écoule ledit liquide condensé, pour récupérer à partir dudit liquide condensé, de l'eau, de l'eau mélangée avec du glycol de concentration inférieure à 99,5 %, et du glycol sensiblement vierge ayant une concentration d'au moins 99,5 %, et
x) orientation dudit glycol sensiblement vierge à partir de son réservoir associé pour des tests supplémentaires et une certification de sa pureté et stockage de celui-ci dans des cuves de rétention certifiées pour une utilisation par des véhicules de dégivrage d'aéronef.

2. Procédé selon la revendication 1, dans lequel après l'étape (x) de certification de pureté et de stockage de glycol sensiblement vierge dans des cuves de rétention certifiées à l'étape (x), il est prévu l'étape supplémentaire de mélange de ladite solution de glycol certifiée d'au moins 99,5 % dans une cuve de mélange dans laquelle des additifs sont mélangés avec ledit glycol sensiblement vierge certifié.

3. Procédé selon la revendication 1, dans lequel ledit glycol sensiblement vierge de l'étape (ix) a une concentration dans la plage allant d'au moins 99,5 % jusqu'à 99,9 %.

4. Procédé selon la revendication 1 dans lequel à l'étape (ix), ladite eau séparée mélangée à du glycol de concentration inférieure à 99,5 % et collectée dans des réservoirs associés est renvoyée dans une cuve de rétention de ladite tour de distillation pour être réintroduite dans ladite tour de distillation pour une évaporation supplémentaire, ladite étape (v) comprenant en outre la fourniture dudit volume prédéterminé de concentration de glycol filtré sur charbon à ladite cuve de rétention de ladite tour de distillation.

5. Procédé selon la revendication 1, dans lequel dans ladite étape (ii) ledit glycol usé ayant une faible concentration prédéterminée inférieure à 5 %, telle que déterminée par un densimètre qui fournit des signaux de concentration de glycol à un dispositif de commande informatique, est amené à monter dans ledit moyen de stockage jusqu'à un conduit de trop-plein pour rejet dans les eaux usées.

6. Procédé selon la revendication 1, dans lequel dans ladite étape de filtration (iii) l'un desdits au moins deux étages de filtration comprend un filtre à manches de 5 microns qui fournit une solution de glycol filtré à une cuve de travail et dans lequel ladite solution de glycol filtré de ladite cuve de travail est pompée dans une boucle continue et à travers un filtre céramique de 0,5 micron, ledit glycol usé fonctionnel étant filtré dans ladite boucle continue pour obtenir du glycol usé filtré souhaité exempt de substances d'une granulométrie supérieure à 0,5 micron, et fournit ledit glycol usé ultrafiltré à une autre cuve de rétention qui fournit ledit glycol usé filtré à un ou plusieurs évaporateurs.

7. Procédé selon la revendication 6, dans lequel ladite autre cuve de rétention est pourvue d'un moyen de détection de niveau pour délivrer des signaux de niveau à un dispositif de commande informatique qui commande le fonctionnement de ladite boucle de filtration continue, lesdits évaporateurs fonctionnant en continu et ayant un moyen de détection de niveau pour permettre audit dispositif de commande informatique de distribuer à ceux-ci une solution de glycol usé filtré afin de maintenir un volume prédéterminé de ladite solution de glycol usé filtré dans lesdits un ou plusieurs évaporateurs.

8. Procédé selon la revendication 7, dans lequel ledit évaporateur est pourvu de deux éléments chauffants résistifs électriques reliés pour un fonctionnement indépendant par l'intermédiaire d'un moyen de commutation actionné par ledit dispositif de commande informatique pour assurer un fonctionnement continu dudit évaporateur en cas de panne de l'un desdits éléments chauffants résistifs, d'un densimètre associé à chacun desdits un ou plusieurs évaporateurs pour détecter la concentration de glycol dans une zone prédéterminée desdits évaporateurs afin de fournir des valeurs de signal de concentration de glycol audit dispositif de commande informatique pour actionner des vannes afin d'évacuer une solution de glycol usé ayant une concentration de 50 % d'une région inférieure desdits un ou plusieurs évaporateurs dans ladite cuve tampon et pour fermer ladite vanne lorsque lesdites valeurs de signal de concentration tombent en dessous de 50 % de concentration de glycol.

9. Procédé selon la revendication 1, dans lequel ladite concentration de glycol d'environ 50 % stockée dans ladite cuve tampon est soumise à un prétraitement tel que défini par l'étape (iv) par l'ajout de NaOH (hydroxyde de sodium) dans ladite cuve d'agitateur à volonté pour ajuster le pH à ladite valeur souhaitée, ladite valeur souhaitée étant d'environ 7,8 sur l'échelle de pH, et en outre dans lequel ladite filtration sur charbon de l'étape (v) consiste à éliminer la couleur et les odeurs de ladite concentration de glycol avant de fournir celle-ci à une cuve de rétention de ladite tour de distillation où un niveau de contrôle de ladite concentration de glycol prétraitée de 50 % est maintenu pour une alimentation par lots d'une section d'évaporateur de ladite tour de distillation.

10. Procédé selon la revendication 1, dans lequel ladite section d'évaporateur de ladite tour de distillation a un serpentin de chauffage dans lequel circule une vapeur chaude provenant d'une chaudière pour évaporer dans ledit flux de vapeurs ledit lot prédéterminé de glycol de concentration de 50 %, une pompe à vide reliée à ladite tour de distillation en aval de ladite section d'évaporateur, ladite section de remplissage chaud ayant une pluralité de remplissages en acier inoxydable qui sont chauffés le long de ladite section de cheminée par ledit flux de vapeurs, et dans lequel après ladite étape (viii) et avant ladite étape (ix) il est prévu l'étape supplémentaire de fourniture dudit liquide condensé à un dispositif de refroidissement pour obtenir une condensation complète.

11. Procédé selon la revendication 10, dans lequel ladite étape (ix) de surveillance est effectuée par un réfractomètre envoyant des signaux audit dispositif de commande informatique qui actionne des vannes pour diriger lesdits liquides condensés vers lesdits réservoirs associés desdits réservoirs.

12. Procédé selon la revendication 2, dans lequel ladite cuve de mélange est pourvue de robinets de niveau pour permettre de tester le pH, la limpidité, le Brix et l'indice de réfraction dudit glycol de dégivrage sensiblement vierge à différents niveaux de ladite cuve de mélange, le volume dudit glycol sensiblement vierge dans ladite cuve de mélange étant déterminé par une échelle disposée sous ladite cuve de mélange, et d'un moyen de pompage pour pomper du glycol sensiblement vierge de ladite cuve de mélange vers lesdites cuves de rétention certifiées.

13. Système de recyclage d'éthylène glycol ou de propylène glycol usé récupéré à partir de solutions de dégivrage d'aéronef contenant du glycol, de l'eau et d'autres substances pour produire du glycol sensiblement vierge, ledit système comprend, en combinaison, un moyen de collecte pour récupérer du glycol usé à partir du tarmac d'une zone de dégivrage d'aéronef, le moyen est en outre prévu pour éliminer le glycol usé ayant une faible concentration en pourcentage prédéterminée dudit moyen de collecte, une ou plusieurs cuves de travail sont également prévues pour stocker du glycol usé ayant une concentration de glycol supérieure à ladite faible concentration en pourcentage prédéterminée, un moyen de filtration est également prévu pour filtrer le glycol usé de la cuve de travail afin d'éliminer sensiblement tous les solides dudit glycol usé, ledit moyen de filtration ayant au moins deux étages de filtration dont l'un est un moyen d'ultrafiltration en boucle, un ou plusieurs évaporateurs pour évaporer l'eau dudit glycol usé afin de produire du glycol usé ayant une concentration de glycol d'environ 50 % qui est ensuite transféré par lots à une cuve tampon, un moyen est prévu pour ajuster le pH du lot de glycol de concentration d'environ 50 % à une valeur de pH souhaitée qui est ensuite filtré sur charbon et fourni à une cuve de rétention de tour de distillation pour accumuler un volume prédéterminé de la concentration de glycol usé de concentration supérieure à environ 50 % pour fournir un lot de glycol usé à une section d'évaporateur d'une tour de distillation qui fonctionne sous vide, une section de cheminée supérieure est pourvue de remplissages en acier inoxydable pour retenir la chaleur au-dessus des températures de condensation du liquide évaporé de ladite section d'évaporateur inférieure, des capteurs de température surveillant la température de ladite section de cheminée pourvue desdits remplissages pour fournir des signaux de température détectés représentatifs de la température d'un flux de vapeurs aspiré à travers lesdits remplissages et fournissant lesdits signaux de température à un dispositif de commande informatique, ladite section d'évaporateur inférieure évapore le glycol usé pour créer ledit flux de vapeurs dont les vapeurs sont aspirées à travers la section de cheminée et dans un serpentin de condensation en aval de ladite section de cheminée pour produire un liquide condensé à partir dudit flux de vapeurs qui est fourni à travers un dispositif de refroidissement ; un moyen de mesure est en outre prévu pour mesurer en continu la concentration de glycol dans le liquide condensé et fournir les signaux de concentration de glycol audit dispositif de commande informatique pour les corréler avec lesdits signaux de température afin de déterminer le moment approprié pour actionner les vannes afin de séparer, dans des réservoirs associés, l'eau, l'eau mélangée avec du glycol contenant du glycol et le glycol sensiblement vierge ayant une concentration de glycol d'au moins 99,5 %, et un moyen d'essai de qualité pour certifier et stocker ledit glycol sensiblement vierge dans des cuves de rétention certifiées pour une utilisation par des véhicules de dégivrage d'aéronef.

14. Système selon la revendication 13, dans lequel ledit moyen d'élimination de glycol usé ayant une faible concentration en pourcentage prédéterminée est constitué par un conduit de trop-plein dans une partie supérieure dudit moyen de collecte, et une pompe adaptée pour pomper du glycol usé d'une région prédéterminée dudit moyen de collecte vers un densimètre pour fournir des signaux de concentration de glycol usé à un dispositif de commande informatique qui détermine si le glycol usé doit être évacué dans ledit conduit de trop-plein ou si le glycol usé ayant une concentration d'au moins 5 % doit être transféré vers lesdites une ou plusieurs cuves de travail.

15. Système selon la revendication 13, dans lequel ledit moyen de filtration comporte un premier étage de filtration pour produire un lot de premier étage de filtration de glycol usé pour la collecte dans une cuve de travail, un second étage d'ultrafiltration fixé dans une boucle à travers laquelle du glycol usé filtré provenant de ladite cuve de travail est mis en circulation en continu pour extraire du glycol usé finement filtré afin d'alimenter une cuve de rétention d'évaporateur.

16. Système selon la revendication 15, dans lequel ledit second étage d'ultrafiltration comporte un filtre céramique ultrafin à travers lequel ledit lot de premier étage de filtration de glycol usé provenant de ladite cuve de rétention est alimenté pour éliminer des solides supplémentaires desdites autres substances supérieurs à 0,5 micron.

17. Système selon la revendication 15, dans lequel un moyen de mélange est en outre prévu pour incorporer des additifs dans ledit glycol sensiblement vierge pour produire du glycol de dégivrage de type I de la plus haute qualité et de concentration supérieure à au moins 99,5 %.

18. Système selon la revendication 17, dans lequel il est en outre prévu une cuve de mélange d'ajustement de pH ayant un moyen d'échantillonnage pour tester le pH dudit glycol usé de concentration de glycol d'environ 50 % et un moyen d'introduction d'additifs dans ladite cuve de mélange afin de produire une valeur de pH souhaitée pour ledit glycol usé de concentration de glycol d'environ 50 % avant de transférer ledit glycol usé de concentration de glycol d'environ 50 % vers une autre cuve de rétention pour fournir des lots dudit glycol usé de concentration de 50 % à ladite section d'évaporateur inférieure de ladite tour de distillation.

19. Système selon la revendication 13, dans lequel ladite concentration de glycol sensiblement vierge séparé d'au moins 99,5 % est de préférence dans la plage allant d'environ 99,6 % à 99,9 % de concentration.

20. Système selon la revendication 13, dans lequel ledit moyen de mesure pour surveiller la concentration en pourcentage de glycol est opéré par un réfractomètre pour mesurer l'indice de réfraction dudit glycol dans ledit liquide de condensation, ledit réfractomètre fournissant des signaux de valeur d'indice de réfraction à un dispositif de commande informatique.

21. Système selon la revendication 20, dans lequel lesdits moyens de séparation sont des vannes qui sont actionnées automatiquement par ledit dispositif de commande informatique sur la base de signaux de valeur d'indice reçus provenant dudit réfractomètre moyennant quoi lesdites vannes sont actionnées afin de diriger ledit liquide de condensation vers lesdits réservoirs associés des réservoirs de stockage.

22. Système selon la revendication 21, dans lequel lesdits réservoirs de stockage sont pourvus de moyens de conduite pour diriger ledit liquide de condensation collecté à travers une vanne vers un emplacement souhaité, ladite eau dans l'un desdits réservoirs de stockage étant dirigée vers le rejet ou la réutilisation, ladite eau mélangée avec du glycol de concentration inférieure à 99,5 % étant redirigée vers ladite autre cuve de rétention alimentant ladite section d'évaporateur de ladite tour de distillation, et ledit glycol sensiblement vierge étant dirigé vers des cuves de stockage de glycol certifiées.

23. Système selon la revendication 22, dans lequel il est en outre prévu une cuve de rétention d'essai de contrôle de qualité pour permettre l'essai de contrôle de qualité pour la certification dudit glycol sensiblement vierge, et une cuve de mélange pour ajouter d'autres additifs audit glycol sensiblement vierge certifié pour le stockage dans des cuves prêtes à l'emploi pour une utilisation par des véhicules adaptés pour mélanger une solution finale de glycol de dégivrage d'aéronef en fonction des facteurs climatiques.
